# EUROPÄISCHE PATENTSCHRIFT

(11) **EP 1 711 213 B1**
(45) Veröffentlichungstag und Bekanntmachung des Hinweises auf die Patenterteilung: **19.11.2014**
(21) Anmeldenummer: 05701357.5
(22) Anmeldetag: 04.02.2005
(51) Int. Cl.: A61L 31/02, A61L 31/14, A61L 31/16

(54) **IMPLANTAT ZUR FREISETZUNG EINES WIRKSTOFFS IN EIN VON EINEM KÖRPERMEDIUM DURCHSTRÖMTES GEFÄSS**
IMPLANT FOR RELEASING AN ACTIVE SUBSTANCE INTO A VESSEL THROUGH WHICH A BODY MEDIUM FLOWS
IMPLANT PERMETTANT DE LIBERER UNE SUBSTANCE ACTIVE DANS UN RECIPIENT TRAVERSE PAR UN FLUIDE CORPOREL

(30) Priorität: 06.02.2004 DE 102004006745; 09.06.2004 DE 102004029611
(43) Veröffentlichungstag der Anmeldung: 18.10.2006
(73) Patentinhaber: Biotronik VI Patent AG, 6341 Baar (CH)
(72) Erfinder: FLACH, Erhard, 12305 Berlin (DE); GEISTERT, Wolfgang, 79618 Rheinfelden (DE); KOLBERG, Gernot, 12043 Berlin (DE); HARDER, Claus, 91080 Uttemreuth (DE); ROHDE, Roland, 31303 Burgdorf (DE); HEUBLEIN, Bernd, deaceased (DE); MÜLLER, Heinz, 91054 Erlangen (DE)
(74) Vertreter: Lindner-Vogt, Karin L.
(86) Internationale Anmeldenummer: PCT/EP2005/001167
(87) Internationale Veröffentlichungsnummer: WO 2005/075005

(56) Entgegenhaltungen:
- EP-A- 0 875 218
- EP-A- 1 270 023
- EP-A- 1 310 266
- EP-A- 1 389 472
- EP-A- 1 419 793
- WO-A-02/100452
- DE-A1- 19 945 049
- US-A- 5 419 760
- US-B1- 6 287 332

## Beschreibung

Die Erfindung betrifft ein Implantat zur Freisetzung eines Wirkstoffs in ein von einem Körpermedium durchströmtes Gefäß sowie Verwendungen eines derartigen Implantats.

Seit Jahrtausenden versucht der Mensch durch gezielte Verabreichung pharmazeutisch wirksamer Substanzen den Heilungsverlaufes pathologischer Vorgänge und Zustände zu beschleunigen bzw. die mit der Krankheit verbundene Symptomatik zu mindern. Neben der Auswahl und Suche nach geeigneten Wirkstoffen, liegt ein Problem darin, den Wirkstoff an dem gewünschten Behandlungsort zur Verfügung zu stellen. Um die häufig mit der Verabreichung einhergehenden Nebenwirkungen gering zu halten, sollte die Freisetzung des Wirkstoffs möglichst nur auf den Ort der Behandlung beschränkt sein. Weiterhin ist es zur Optimierung der Wirkung oftmals notwendig eine Dosierung möglichst genau einzuhalten, d. h. die Wirkstoffkonzentration über einen vorgebbaren Zeitraum am Behandlungsort in bestimmten Bereichsgrenzen zu halten. Herkömmlicherweise werden Wirkstoffe oral, subkutan, intravenös oder rektal appliziert. Gerade bei lokalen Erkrankungen, wie z. B. Tumoren, führt die konventionelle systemische Medikamentengabe zu erheblichen Komplikationen.

Seit einigen Jahren bestehen daher vermehrt Bestrebungen, den Wirkstoff gezielter in den Körper des zu behandelnden Patienten einzubringen. Für eine lediglich lokale Behandlung von einem Implantat aus, d.h. Elution eines Wirkstoffes im Wesentlichen nur in das das Implantat unmittelbar umgebende Gewebe, hat sich der Begriff 'Local Drug Delivery' (LDD) etabliert. Der Behandlungsort, an dem der Wirkstoff seine pharmakologische Wirkung entfalten soll, grenzt demnach unmittelbar an den Implantationsort.

Ein wichtiger Anwendungsbereich für LDD-Systeme liegt beispielsweise in der Verbesserung der Biokompatibilität von Permanentimplantaten, wie Stents, Herzschrittmachern oder orthopädischen Prothesen. Hier sollen insbesondere die durch Anwesenheit des Implantats und durch die Implantation verursachten Komplikationen gemindert bzw. vermieden werden.

Eine von LDD-Systemen im vorgenannten Sinne abweichende Wirkstoffdarreichung - der auch die vorliegende Erfindung zuzuordnen ist - liegt der Gedanke zugrunde, Implantationsort und Behandlungsort räumlich stärker voneinander zutrennen. Mit anderen Worten, der am Implantat freigesetzte Wirkstoff soll nicht unmittelbar (lokal) wirken, sondern nach Transport im Körper erst in einer davon räumlich entfernt liegenden Gewebsregion seine Wirkung entfalten. Für diese Art der regionalen Wirkstoffdarreichung wir im folgenden der Begriff 'Regional Drug Delivery' - oder kurz RDD - verwendet.

Bei bestimmten Krankheitsbildern, z.B. lokal begrenzten Tumoren, bietet sich eine Wirkstoffdarreichung über das vaskuläre System des zu behandelnden Gewebes an. Dazu ist es notwendig, das Implantat in ein dem Behandlungsort vorgelagertes Gefäß einzubringen. Ein Ansatz beim Stand der Technik besteht beispielsweise darin, eine polymere Matrix, die den Wirkstoff enthält, in das Gefäß zu injizieren. Die Matrix ist dabei derart beschaffen, dass sie sich unmittelbar nach Injektion zu einem zähen Film wandelt, der an den Gefäßwänden haftet. Dieser den Wirkstoff enthaltende Film zersetzt sich allmählich, wobei der Wirkstoff freigesetzt wird. Es ist allerdings in der Praxis sehr schwierig, eine derart punktgenaue Injektion in das betreffende Gefäß zu erreichen. Weiterhin kommt es in jedem Fall zu einer Verletzung der Gefäßwand, was zum Beispiel durch Thrombusbildung zu weiteren Komplikationen führen kann. Schließlich hängt eine Dosierung auch von der Dicke des Films ab, d. h. es ist nur schwer vorhersagbar, welche Freisetzungscharakteristik tatsächlich besteht.

Der vorliegenden Erfindung liegt daher die Aufgabe zugrunde, ein Implantat für die Freisetzung eines Wirkstoffs in einem von einem Körpermedium durchströmten Gefäß bereitzustellen.

Diese Aufgabe wird durch das Implantat mit den in dem Anspruch 1 genannten Merkmalen gelöst. Das Implantat zur Freisetzung eines Wirkstoffs in einem von einem Körpermedium durchströmten Gefäß zeichnet sich dadurch aus, dass es einen Grundkörper umfasst, der aus einem biodegradierbaren Material als Träger des freizusetzenden Wirkstoffs besteht und der von dem Körpermedium innen umströmt wird. Mit anderen Worten, das Körpermedium, insbesondere Blut, strömt weitestgehend ungehindert durch das Implantat nach dessen Implantation. Der Grundkörper des Implantats dient als Träger für den Wirkstoff, der zumindest weitestgehend in das vorbeiströmende Körpermedium eluiert und an einen nachgeordneten Behandlungsort verbracht wird. Der Grundkörper des Implantats wird im Laufe der Zeit abgebaut.

Das Implantat eignet sich demnach besonders für die Zwecke der regionalen Wirkstoffdarreichung (RDD), insbesondere zur Behandlung von Tumoren. Daher werden diese Verwendungen des Implantats gesondert beansprucht.

Unter Gefäß im Sinne der Erfindung wird die Gesamtheit der arteriellen und venösen Blutgefäße einschließlich der Gefäße der Endstrombahn (i. w. S. auch der Lymphgefäße), die zusammen mit dem Herzen eine funktionelle Einheit bilden, verstanden. Das erfindungsgemäße Implantat ist dazu ausgebildet, den Wirkstoff im Körpermedium freizusetzen, das das Gefäß durchströmt. Auf diese Weise gelangt der Wirkstoff vom Freisetzungsort an den eigentlichen, weiter stromab gelegenen Wirkort. Das Implantat ist demnach so auszubilden, dass der Wirkstoff überwiegend - vorzugsweise mindestens 80 Gew.% des eingesetzten Wirkstoffs - im Innern des Gefäßes freigesetzt wird und vom Körpermedium mitgenommen wird. Eine Freisetzung in Richtung der Gefäßwand ist zu vermeiden. Demnach agieren bei den erfindungsgemäßen Implantaten vorzugsweise die Bereiche, die nach der Implantation mit einer Gefäßwand in Kontakt stehen, nicht als Träger des Wirkstoffs. Hat das Implantat z. B. eine rohrförmige Kontur, die sich nach Implantation mit ihrer Außenwandung am Gefäß abstützt, so ist auf der Außenwandung kein Wirkstoff aufgebracht.

Unter Grundkörper im erfindungsgemäßen Sinne wird eine Struktur des Implantats verstanden, die vor Beginn der Biodegradation die mechanische Integrität des Implantats gewährleistet und die als Träger des freizusetzenden Wirkstoffs oder einer den Wirkstoff beinhaltenden Matrix dient.

EP 1 310 266 A1 offenbart einen vaskulären Stent zur Freisetzung eines Wirkstoffs in ein von einem Körpermedium durchströmtes Gefäß, der aus einem biodegradierbaren Material mit Poren/Kavitäten gefüllt mit einem Wirkstoff in einer biodegradierbaren Matrix besteht. Der Stent wird von dem Körpermedium innen umströmt, so dass der Wirkstoff in den Blutstrom abgegeben wird.
Strömt das Körpermedium nur durch den Grundkörper, so ist dieser vorzugsweise als rohrförmiger, an seinen Endseiten offener Grundkörper ausgebildet, der (im implantierten Zustand) mit seiner Außenseite an der Gefäßwand anliegt. Aufgrund der an den Gefäßquerschnitt angepassten Formgebung werden Turbulenzen im Körpermedium weitgehend unterdrückt oder zumindest gemindert, so dass sich diese Implantatsvariante insbesondere für Gefäße mit einem hohen Volumenstrom an Körpermedium eignet.

Nach einer zweiten Variante des Implantats, die nicht Gegenstand der Patentanmeldung ist, strömt das Körpermedium sowohl innen als auch außen durch bzw. um den Grundkörper. Der Grundkörper kann dazu als Hohlkörper ausgelegt sein, der in Strömungsrichtung ausgerichtete Ein- und Auslässe aufweist. Diese für das Körpermedium unmittelbar oder kurz nach Implantation zugänglichen Hohlkörper können insbesondere rohrförmig, zylindrisch oder kugelförmig ausgestaltet sein. Zur Sicherung der relativen Lage des Implantats im Körper sind zumindest weitgehend biodegradierbare Ankerelemente vorhanden, die sich vom Grundkörper ausgehend bis zur Gefäßwand erstrecken und dort der Verankerung dienen.

Die Ankerelemente können beispielsweise eine zacken-, haken- oder schuppenförmige Kontur besitzen. Die Ankerelemente sind so auszulegen, dass sie eine Verankerung des Implantats zumindest über den Freisetzungszeitraum für 90 Gew.%, insbesondere 95 Gew.% des Wirkstoffs sicherstellen. Der Einsatz von Ankerelementen verringert die Kontaktfläche zwischen dem Implantat und der Gefäßwand, so dass der Bereich einer möglichen Endotheliasierung ebenfalls begrenzt wird. Eine konkrete Ausgestaltung der Ankerelemente hängt u.a. von den am Implantationsort herrschenden Strömungsverhältnisses, dem Freisetzungsverhalten des Wirkstoffs und der Degradationscharakteristik des Grundkörpers ab, so dass eine individuelle Adaption des Implantats an die jeweilige Applikation zu erfolgen hat. Der Fachmann wird dabei zur Umsetzung dieses Ziels den allgemeinen Erkenntnissen
- zur Rheologie von Körpergefäßen,
- zur Beeinflussung des Degradationsverhalten der biodegradierbaren Materialien durch stoffliche Modifikation oder Legierungswahl, inklusive deren Verarbeitung und Beschichtung sowie
- zur Beeinflussung des Freisetzungsverhaltens von Wirkstoffen in Abhängigkeit seiner Modifikation oder Einbindung in eine Matrix
folge leisten können. Aus fertigungstechnischen Gründen sind die Ankerelemente vorzugsweise aus demselben biodegradierbaren Material geformt und mit dem Grundkörper einteilig verbunden.

Eine dritte Variante des Implantats, die ebenfalls nicht Gegenstand der vorliegenden Patentanmeldung ist, sieht vor, dass der Grundkörper nur von dem Körpermedium umströmt wird. Mit anderen Worten, der Grundkörper zeigt eine geschlossene Struktur, bei der nur die Außenwandungen des Grundkörpers mit dem Körpermedium in Kontakt treten. Der Grundkörper ist entweder kompakt ausgebildet oder die bei hohlen Grundkörpern vorhandenen Innenwandungen des Grundkörpers werden erst durch Biodegradation zugänglich. Denkbar sind insbesondere Grundkörper mit netzartigen, kegelförmigen oder lamellenförmigen Grundmuster. Die Grundkörper dieser Implantatsvariante können wiederum mit Hilfe der zuvor beschriebenen Ankerelemente im Gefäß fixiert werden.

Eine bevorzugte Variante der vorangehend beschriebenen geschlossenen Struktur sieht vor, dass der Grundkörper von Außen nach Innen mehrschichtig aufgebaut ist. Auf den beziehungsweise zwischen den Schichten befindet sich der freizusetzende Wirkstoff. Im Körper wird zunächst die äußere Schicht abgebaut und der darunter liegende Wirkstoff freigesetzt. Dann wird die nächste Schicht abgebaut und der unter dieser Schicht befindliche Wirkstoff freigesetzt, und so weiter.

Nach einer bevorzugten Ausführungsform der Erfindung besteht der Grundkörper des Implantats, zumindest bereichsweise, aus einer biodegradierbaren Magnesium-, Eisen- oder Wolframlegierung. In diesen Legierungen hat das namensgebende Element jeweils mindestens einen Anteil an der Legierung von 50 Gew.%, insbesondere 70 Gew.%, besonders bevorzugt 80 Gew.%. Ein Implantat in Form eines Stents bestehend aus einer oben genannten Wolframlegierung ist beispielsweise aus der DE 199 45 049 bekannt.
Besonders bevorzugt sind ferner Magnesiumlegierungen vom Typ WE, insbesondere WE43, bei denen Seltenerdmetalle und Yttrium der Legierung zugesetzt sind. Die genannten Legierungen lassen sich technisch gut verarbeiten, haben für die Realisation der erfindungsgemäßen Implantate besonders geeignete mechanische Werkstoffeigenschaften und zeigen ein günstiges Abbauverhalten im lebenden Organismus. Gerade bei den Magnesiumlegierungen scheint zudem ein positiver physiologischer Effekt bei der Biodegradation des Grundkörpers auf das umgebende Gewebe aufzutreten.

Ferner sind Magnesiumlegierungen, die einen Gehalt von 1 und 30 Gew.% Lithium enthalten, wegen ihrer zu erwartenden hohen Biokompatibilität bevorzugt. Weiterhin sind Magnesiumlegierungen mit einem Gehalt von 0,1 Gew.% bis 10 Gew.% Aluminium sowie Magnesiumlegierungen mit einem Gehalt von 0,01 Gew.% bis 2 Gew.% Zirkonium wegen ihrer verarbeitungstechnischen, mechanischen und abbaurelevanten Eigenschaften besonders bevorzugt. Die genannten Bestandteile der Magnesiumlegierung - nämlich Seltenerdmetalle (E), Yttrium (W), Lithium (L), Aluminium (A) und Zirkonium (K) - können in beliebiger Kombination Bestandteil der Legierung sein, wobei in Klammern die normierten Kurzbezeichnungen der Legierungsbestandteile nach ASTM angegeben sind. So können beispielsweise Legierungszusammensetzungen folgenden Typs verwendet werden: LWE, AL, LAE und LE, wobei die Letternfolge je nach verwendeter Legierungszusammensetzung auch permutiert sein kann. Die Magnesiumlegierung enthält demnach vorzugsweise ein oder mehrere Legierungsbestandteile aus der Gruppe Seltenerdmetalle, Yttrium, Lithium, Aluminium und Zirkonium.

Der Grundkörper des Implantats wird ferner vorzugsweise derart ausgestaltet, dass er einen ersten, nicht-expandierten Zustand und einen zweiten, expandierten Zustand einnehmen kann. Zur Realisation derartiger Strukturen kann auf die zahlreichen bekannten Designs von Stents zurückgegriffen werden. Anzumerken ist jedoch, dass das erfindungsgemäße Implantat dieser Variante im Gegensatz zu Stents keinerlei Stützfunktion für die Gefäßwand auszuüben braucht bzw. soll. Es muss lediglich sichergestellt sein, dass sich das Implantat in dem Gefäß verankert, d. h. nicht durch den stetigen Strom des Körpermediums mitgerissen wird. Vom Design bestehen daher größere Freiheiten als bei Implantaten mit Stützfunktion. Ist beispielsweise der Wirkstoff Bestandteil einer den Grundkörper bedeckenden Beschichtung, so sollten die dem Körpermedium zugewandten Teilbereiche des Grundkörpers möglichst großflächig ausgelegt und vom Wirkstoff bedeckt sein. Eine Endotheliasierung, d.h. ein Einwachsen des Implantats kann in soweit toleriert werden, als dass die Elution des Wirkstoffs nicht beeinträchtigt wird. Gegebenenfalls muss z. B. durch bestimmte Oberflächenstrukturen auf der luminalen Seite des Implantats oder Beschichtung mit dem Prozess des Einwachsens entgegenwirkenden Substanzen gegengesteuert werden.

Vorzugsweise ist das erfindungsgemäße Implantat zur Freisetzung eines Wirkstoffs in ein von einem Körpermedium durchströmtes Gefäß wie folgt beschaffen:
- Der Grundkörper weist an seinen dem Gefäß zugewandten Seiten zumindest bereichsweise eine Beschichtung auf, die den Wirkstoff enthält,
- der Grundkörper weist ein oder mehrere Kavitäten auf, die den Wirkstoff enthalten und/oder
- der Grundkörper weist ein oder mehrere Hohlkörper auf, die den Wirkstoff enthalten.

Eine erste Variante sieht demnach vor, dass der Grundkörper zumindest teilweise mit dem Wirkstoff beschichtet ist. Die Beschichtung kann dabei aus dem Wirkstoff selbst, aber auch aus einer den Wirkstoff enthaltenden, biodegradierbaren Matrix bestehen. Denkbar ist beispielsweise, dass der Wirkstoff in eine Matrix aus Hyaluronsäure oder seinen Derivaten eingebettet ist. Die Wahl der Matrix, aber auch die Darreichungsform des Wirkstoffs beeinflusst stark das in vivo Freisetzungsverhalten des Wirkstoffs. Eine Optimierung des Freisetzungsverhaltens kann aufgrund der sehr unterschiedlichen, das Freisetzungsverhalten beeinflussenden Größen nur am konkreten System erarbeitet werden. Anzumerken bleibt, dass der Ort der Beschichtung vorzugsweise so vorzugeben ist, dass der Wirkstoff nahezu vollständig in das durch das Gefäß strömende Körpermedium und nicht in Richtung der anliegenden Gefäßwand abgegeben wird.

Nach einer zweiten Variante können Kavitäten, die den Wirkstoff enthalten, im Grundkörper eingearbeitet sein. Unter Kavitäten im Sinne der Erfindung werden Vertiefungen, Spalten oder auch Lochbohrungen in den Grundkörper des Implantats verstanden, die nicht vollständig von dem Grundkörper umschlossen sind, d. h. zumindest an einer Seite zugänglich sind. Der Wirkstoff befindet sich entweder in Reinform oder in eine Matrix eingebunden im Inneren der Kavität. Die Erzeugung derartiger Kavitäten ist - z. B. aus dem Bereich Stents - hinlänglich bekannt und kann beispielsweise mit Hilfe von Laserverfahren durchgeführt werden. Festzuhalten bleibt lediglich, dass das Körpermedium, welches durch oder um das erfindungsgemäße Implantat strömt, allmählich den Wirkstoff aus den Kavitäten herauslöst. Dieser Prozess beschleunigt sich mit fortschreitender Degradation des Grundkörpers. Gegenüber der Beschichtung ist in der Regel mit einer verzögerten Freisetzung zu rechnen.

Schließlich sieht eine weitere Variante des erfindungsgemäßen Implantats vor, dass der Grundkörper einen oder mehrere Hohlkörper enthält, in die der Wirkstoff eingebracht ist. Auch hier kann der Wirkstoff entweder als Reinsubstanz oder aber eingebettet in einer Matrix vorliegen. Hohlräume im Sinne der Erfindung sind vom Grundkörper vollständig umschlossene Räume, in die der Wirkstoff zuvor eingebracht wurde. Erst durch die allmähliche Degradation des Grundkörpers wird der Wirkstoff zugänglich und kann sich im vorbei strömenden Körpermedium lösen. Dementsprechend zeigt die vorliegende Variante im allgemeinen eine gegenüber den beiden zuvor geschilderten Varianten am stärksten verzögerte Wirkstofffreisetzung. Eine denkbare Variante sieht beispielsweise vor, den Grundkörper aus einem hohlen Draht zu formen, dessen Inneres mit dem Wirkstoff befüllt ist.

Alle drei vorgenannten Varianten lassen sich in beliebiger Weise kombinieren, sei es, um das Freisetzungsprofil eines einzelnen Wirkstoff zu beeinflussen, oder sei es, um die Freisetzung verschiedener Wirkstoffe in vorgebbarer zeitlicher Abfolge zu steuern.

Die Implantate lassen sich problemlos mit an bekannten Techniken angelehnten Implantationssystemen in ein von einem Körpermedium durchströmtes Gefäß einbringen. So kann vorgesehen sein, dass das Implantat auf einem Ballon eines Ballonkathetersystems montiert wird. Der Katheter wird dann in bekannter Manier bis zum Implantationsort geführt. Anschließend wird der das Implantat tragende Ballon expandiert und das Implantat platziert. Nach Deflation des Ballons wird der Katheter zurückgezogen und das Implantat verbleibt in fixierter Position am gewünschten Ort bis es zersetzt ist. Für die Zwecke der Einbringung des erfindungsgemäßem Implantats kann es vorteilhaft sein, wenn das Implantat einen dritten, gecrimpten Zustand auf dem Trägersystem einnehmen kann.

Ein dazu alternatives Implantationssystem kann aus mehreren, insbesondere drei oder vier, langgestreckten Nitinoldrähten bestehen, die sich unter lokal-thermischem Einfluss ausdehnen. Die Nitinoldrähte sind derart zum Implantat angeordnet, dass durch die Ausdehnung derselben eine Verankerung des Implantats am Implantationsort erzwungen werden kann. Das Implantationssystem eignet demnach besonders für Implantate, die die obig geschilderten Ankerelemente beinhalten.

Die Erfindung wird nachfolgend anhand von Ausführungsbeispielen und den dazugehörigen Zeichnungen näher erläutert. Es zeigen:
- Fig. 1a bis 1c: schematische Darstellungen erfindungsgemäßer Implantate zur Freisetzung eines Wirkstoffs in ein von einem Körpermedium durchströmtes Gefäß und
- Fig. 2a bis 2c: schematische Schnittansichten durch Teilbereiche eines Implantats gemäß Figur 1a, 1b oder 1c in verschiedenen Varianten.

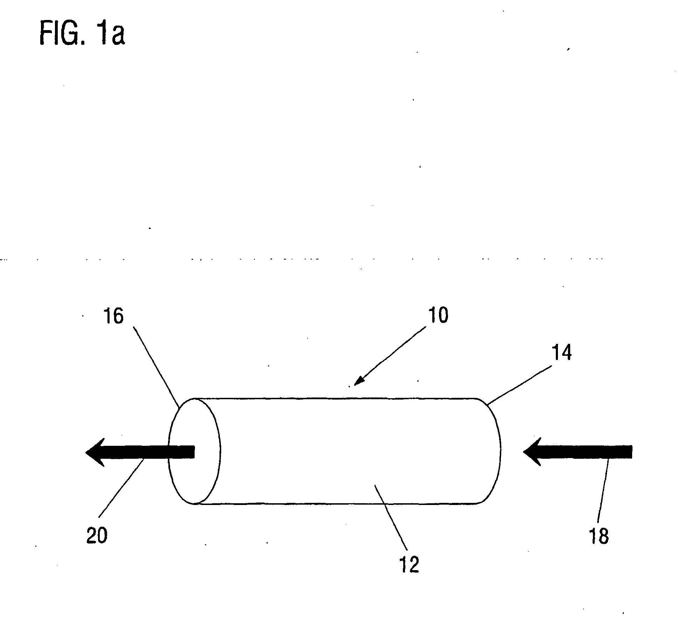

Die Figuren 1a bis 1c zeigen in stark schematisierter Weise Implantate 10, 30, 40, die zur Freisetzung von Wirkstoffen in ein von einem Körpermedium durchströmtes Gefäß geeignet sind.

Der Figur 1a ist eine erste Variante eines erfindungsgemäßen Implantats 10 zu entnehmen. Das Implantat 10 besteht aus einem rohrförmigen Grundkörper 12, der an seinen Stirnseiten 14 und 16 offen ist und der von dem Körpermedium durchströmt werden kann. Eine Strömungsrichtung des durch den Grundkörper 12 des Implantats 10 strömenden Körpermediums, insbesondere Blut, wird durch die beiden Pfeile 18, 20 angedeutet.

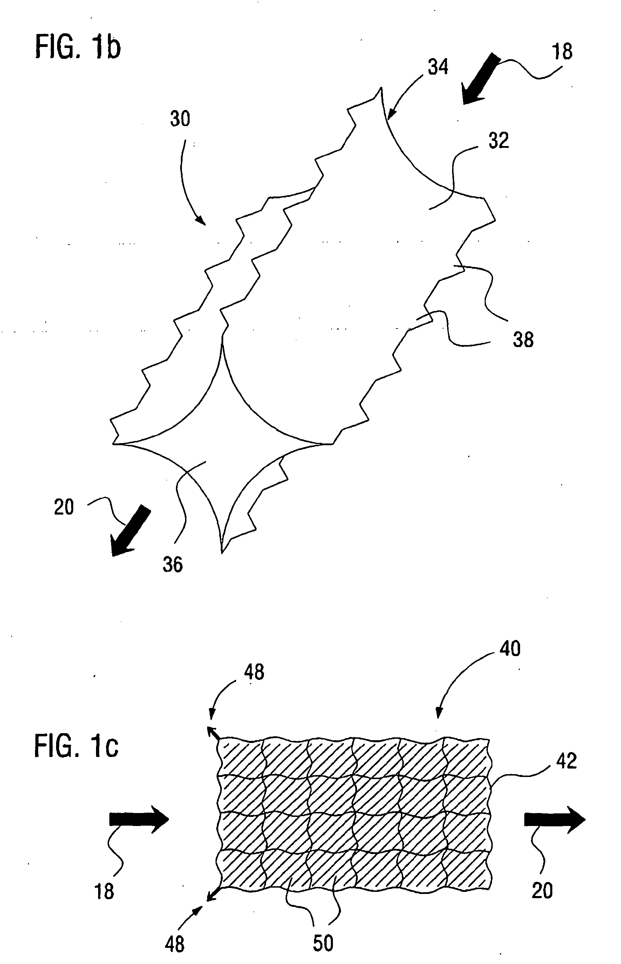

Die Figur 1b zeigt eine zweite Variante eines geeigneten Implantats 30, die nicht Gegenstand der Patentanmeldung ist. Der Grundkörper 32 ist als Hohlkörper ausgelegt, aber nicht in seiner Kontur dem Gefäß, in dem das Implantat 30 verankert werden soll, angepasst. In Strömungsrichtung - wiederum angedeutet durch die Pfeile 18, 20 - weist der Grundkörper Ein- und Auslässe 34, 36 auf. Das Körpermedium strömt im Gegensatz zur Variante der Figur 1a sowohl innen als auch außen durch bzw. um den Grundkörper 32. Zur Sicherung der relativen Lage des Implantats 32 im Körper sind an den Grundkörper Ankerelemente 38 angeformt, die sich nach der Implantation an der Gefäßwand abstützen und aufgrund ihrer Formgebung einen Halt gewähren. Die Ankerelemente 38 sind im konkreten Fall zackenförmig ausgebildet, können jedoch auch andere Konturen annehmen. Ferner bestehen die Ankerelemente 38 zumindest weitestgehend aus einem biodegradierbaren Material. Die Ankerelemente 38 sind so ausgelegt, dass eine Verankerung des Implantats 30 zumindest über den Freisetzungszeitraum von 90 Gew.%, insbesondere 95 Gew.% des Wirkstoffs sicherstellt ist. Eine konkrete Ausgestaltung der Ankerelemente 38 hängt u.a. von den am Implantationsort herrschenden Strömungsverhältnisses, dem Freisetzungsverhalten des Wirkstoffs und der Degradationscharakteristik des Grundkörpers 32 ab, so dass eine individuelle Adaption des Implantats 30 an die jeweilige Applikation zu erfolgen hat. Aus fertigungstechnischen Gründen sind die Ankerelemente 38 vorzugsweise aus demselben biodegradierbaren Material wie der Grundkörper 32 geformt und mit demselben einteilig verbunden.

Figur 1c zeigt schematisch eine dritte Variante des erfindungsgemäßen Implantats 40, die nicht Gegenstand der Patentanmeldung ist. Nach dieser Variante ist der Grundkörper 42 als eine geschlossene Struktur ausgebildet, d.h. nur die Außenwandungen des Grundkörpers treten unmittelbar nach der Implantation mit dem Körpermedium in Kontakt. Der dargestellte Grundkörper 42 hat eine netzartige Struktur und wird mit Hilfe von Ankerelementen 48 im Gefäß fixiert. Die sich zwisehen den einzelnen Fäden des Netzes ergebenden Freiräume 50 werden von einer folienartigen Matrix überspannt, die den Wirkstoff enthält. Die Matrix kann beispielsweise durch Eintaschen des netzartigen Grundkörpers 42 in eine die Matrix enthaltende Lösung und anschließendes Trocknen des benetzten Grundkörpers 42 aufgebracht werden. Der Grundkörper 42 als auch die Matrix sind aus einem biodegradierbaren Material geformt.

Der Grundkörper 12, 32, 42 der Figuren 1a, 1b und 1c dient als Träger für einen, gegebenenfalls auch mehrere im Körpermedium freizusetzende Wirkstoffe. Während oder zumindest nach der Freisetzung des Wirkstoffs zersetzt sich der Grundkörper 12, 32, 42 weitestgehend vollständig, d. h. er besteht zumindest weitestgehend aus einem biodegradierbaren Material.

Als biodegradierbares Material für den Grundkörper 12, 32, 42 kommen insbesondere Legierungen der Elemente Magnesium, Eisen und Wolfram in Frage. Dabei weisen die genannten Elemente an den Legierungen jeweils Anteile von mindestens 50 Gew.%, bevorzugt mehr als 70 Gew.%, besonders bevorzugt mehr als 80 Gew.% auf. Besonders bevorzugt sind Magnesiumlegierungen, die Seltenerdmetalle und Yttrium enthalten, üblicherweise bezeichnet als Legierungen vom Typ WE. Unter letzteren hat sich der Werkstoff WE43 als besonders geeignet erwiesen, d. h. die Biodegradation des Wirkstoffs läuft kontrolliert ab, die bei der Degradation freigesetzten Abbauprodukte haben keinerlei oder allenfalls einen geringen toxischen Effekt und bei der Verarbeitung des Werkstoffs kann auf herkömmliche Bearbeitungstechniken für Magnesiumlegierungen zurückgegriffen werden.

Ferner sind Magnesiumlegierungen, die einen Gehalt von 1 und 30 Gew.% Lithium enthalten, wegen ihrer zu erwartenden hohen Biokompatibilität bevorzugt. Weiterhin sind Magnesiumlegierungen mit einem Gehalt von 0,1 Gew.% bis 10 Gew.% Aluminium sowie Magnesiumlegierungen mit einem Gehalt von 0,01 Gew.% bis 2 Gew.% Zirkonium wegen ihrer verarbeitungstechnischen, mechanischen und abbaurelevanten Eigenschaften besonders bevorzugt. Die genannten Bestandteile der Magnesiumlegierung - nämlich Seltenerdmetalle (E), Yttrium (W), Lithium (L), Aluminium (A) und Zirkonium (K) - können in beliebiger Kombination Bestandteil der Legierung sein, wobei in Klammern die normierten Kurzbezeichnungen der Legierungsbestandteile nach ASTM angegeben sind. So können beispielsweise Legierungszumensetzungen folgenden Typs verwendet werden: LWE, AL, LAE und LE, wobei die Letternfolge je nach verwendeter Legierungszusammensetzung auch permutiert sein kann.

Der Grundkörper 12 des Implantats 10 ist in der Figur 1a nicht näher strukturiert dargestellt. Üblicherweise liegt der Grundkörper 12 jedoch nicht als ein vollständig geschlossenes Rohr vor, sondern setzt sich vielmehr aus einer Vielzahl strebenoder drahtförmiger Strukturelemente zusammen. Ein solcher Aufbau ist besonders deshalb bevorzugt, weil damit die Einbringung des Implantats an den Implantationsort stark erleichtert wird. So kann bei entsprechender Ausgestaltung der Strukturelemente der Grundkörper 12 einen ersten, im Durchmesser geringeren, nicht-expandierten Zustand einnehmen und nach Aufweitung am Implantationsort einen zweiten, expandierten Zustand annehmen. Im nicht-expandierten Zustand des Implantats 10 ist verständlicherweise die Einführung des Implantats bis zum Implantationsort deutlich erleichtert. Dazu kann vorgesehen sein, dass das Implantat 10 auf einen Ballon eines Kathetersystems montiert wird. Der Ballonkatheter wird dann - ganz analog zur Dilatation von Stents mit gleichartigen Systemen - in den Körper eingeführt und an der gewünschten Stelle wird durch Inflation des Ballons das Implantat 10 expandiert. Für die Zwecke der Einbringung des Implantats 10 kann es vorteilhaft sein, wenn das Implantat 10 einen dritten, gecrimpten Zustand auf dem Trägersystem einnehmen kann. Ferner können mit mechanischen Zug- oder Schubvorrichtungen oder mit Hilfe von thermischen Verformungen arbeitende Implantationssysteme zur Anwendung kommen. So können beispielsweise drei oder vier, langgestreckte Nitinoldrähten vorgesehen sein, die sich unter lokal-thermischem Einfluss ausdehnen. Die Nitinoldrähte sind derart zum Implantat angeordnet, dass durch die Ausdehnung derselben eine Verankerung des Implantats am Implantationsort erzwingen. Das Implantationssystem eignet demnach besonders für Implantate, die die obig geschilderten Ankerelemente 38, 48 beinhalten.

Prinzipiell kann das Design der den Grundkörper 12 bildenden Strukturelemente eines Implantats 10 nach Figur 1a an bereits bekannte Stent-Designs angelehnt sein. Festzuhalten ist jedoch, dass das Implantat 10 keine Stützfunktion auszuüben braucht, d. h., dass der Stent sehr weich ausgelegt sein sollte, um Gefäßverletzungen vorzubeugen. Das Design dient der Verankerung im Gefäß und soll einem Mitreißen des Implantats 10 durch das Körpermedium vorbeugen. Die Elution des Wirkstoffs soll ferner nicht durch ein Einwachsen des Implantats 10 in die Gefäßwand behindert werden. Das Design kann daher zu diesem Zwecke angepasst werden, z. B. durch Oberflächenmodifikationen oder Beschichtungen mit antiproliferativen Substanzen an Stellen des Implantats 10, die mit der Gefäßwand in Berührung stehen.

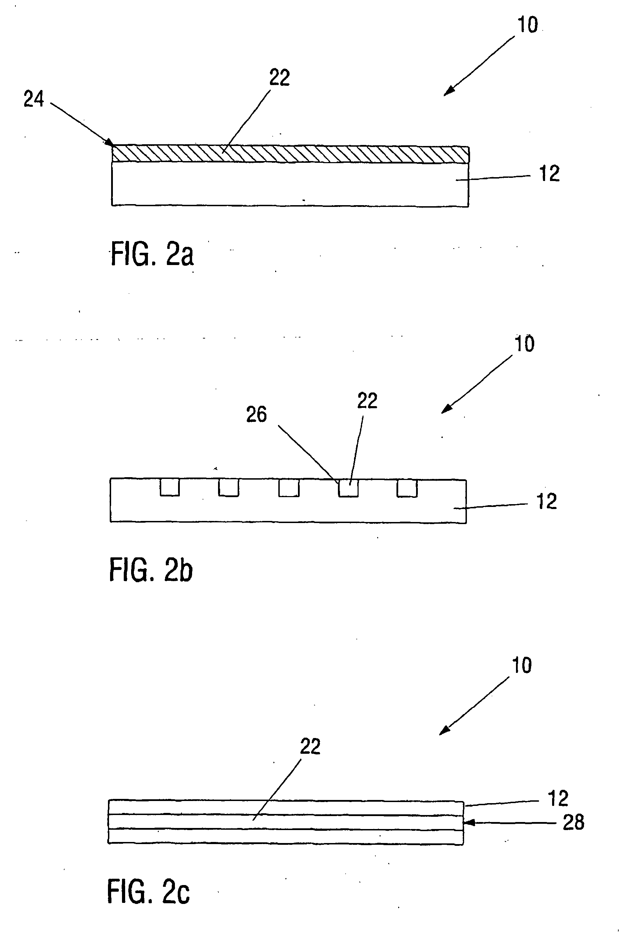

Die Figuren 2a bis 2c zeigen in stark schematisierter Weise Schnitte durch die Implantate 10, 30, 40 gemäß den Figur 1a bis 1c, und zwar jeweils in einem Bereich, in dem der Grundkörper 12, 32, 42 als Träger eines Wirkstoffes 22 oder einer den Wirkstoff 22 enthaltenden Matrix agiert. Der Einfachheit halber wird nachfolgend nur Bezug auf die Variante des Implantats 10 nach Figur 1 a genommen. Die aufgezeigten Maßnahmen lassen sich jedoch ohne weiteres auf andere Implantatsvarianten mit hiervon abweichender Formgebung übertragen.

In der Figur 2a ist der Wirkstoff 22 als Beschichtung 24 auf dem Grundkörper 12 aufgebracht. Sinnvollerweise liegt die Beschichtung 24 auf einer Innenseite des rohrförmigenrohrförmigen Grundkörpers 12, so dass das strömende Körpermedium in Kontakt mit dem Wirkstoff 22 treten kann und diesen in gelöster Form in die nachgeordneten Gewebsbereichen zu eluieren vermag. Der Wirkstoff 22 kann in Reinform, beispielsweise als mikrokristallines oder amorphes Produkt, an dem Grundkörper 12 anhaften. Denkbar ist aber auch, dass der Wirkstoff 22 in eine Matrix eingebettet ist, um beispielsweise ein besseres Anhaften auf dem Grundkörper 12 zu ermöglichen und/oder die Freisetzung des Wirkstoffs 22 zu beeinflussen. Als Matrix kommt beispielsweise ein biodegradierbares Polymer wie Hyaluronsäure und seine Derivate in Frage.

Eine zweite Variante, die in Figur 2b dargestellt ist, sieht vor, Kavitäten 26 in den Grundkörper 12 des Implantats 10 einzubringen und diese mit dem Wirkstoff 22 oder einer den Wirkstoff 22 enthaltenen Matrix zu befüllen. Derartige Kavitäten 26 können beispielsweise mittels bekannter Laserverfahren erzeugt werden. Die Kavitäten 26 können in Form von Spalten, Bohrungen oder andere Geometrien verwirklicht werden. Der Wirkstoff 22 kann in die Kavitäten 26, beispielsweise durch Eintauchen in eine den Wirkstoff 22 enthaltene Lösung, Trocknen der Lösung und Abblasen der außerhalb der Kavitäten 26 vorhandenen Wirkstoffablagerungen definiert eingebracht werden. Durch das vorbeiströmende Körpermedium wird der Wirkstoff 22 langsam aus den Kavitäten 26 gespült. Gleichzeitig wird dieser Prozess durch die allmähliche Degradation des Grundkörpers 12 forciert.

Eine dritte Variante des Implantats 10, die in Figur 2c dargestellt ist, sieht schließlich vor, dass der Grundkörper 12 einen Hohlraum 28 aufweist, in den der Wirkstoff 22 eingebracht ist. Erst nachdem die dem Körpermedium zugewandte Seite des Grundkörpers 12 abgebaut ist wird der Wirkstoff 22 in das Körpermedium eluiert. Ein solches System lässt sich beispielsweise durch Verwendung von Hohldrähten, in die durch Kapillarkräfte Lösungen des Wirkstoffs 22 eingebracht wurden, realisieren.

Es versteht sich von selbst, dass die genannten Maßnahmen zur Steuerung des Freisetzungsvermögens des Wirkstoffs 22 variiert werden können. Es ist zudem denkbar, dass nicht nur ein Wirkstoff, sondern mehrere Wirkstoffe und zwar auch zeitlich zueinander versetzt, freigesetzt werden können. Die Freisetzung des Wirkstoffs bzw. der Wirkstoffe hängt dabei von der Wahl des Trägermaterials, der geometrischen Lage des Wirkstoffs auf dem Grundkörper (d. h., Beschichtung, Kavität oder Hohlraum), den Eigenschaften der möglicherweise den Wirkstoff enthaltenen Matrix sowie den rheologischen und anatomischen Gegebenheiten am Ort der Implantation ab. Die zuvor beschrieben Varianten des erfindungsgemäßem Implantats eignen sich besonders für die Zwecke der Regional Drug Delivery (RDD).

## Patentansprüche

1. Implantat (10) zur Freisetzung eines Wirkstoffs (22) in ein von einem Körpermedium durchströmtes Gefäß, wobei das Implantat (10) einen Grundkörper (12) umfasst, der aus einem biodegradierbarem Material als Träger des im Gefäßinneren freizusetzenden und vom Körpermedium mitzunehmenden Wirkstoffs (22) besteht und der von dem Körpermedium innen umströmt wird,
**dadurch gekennzeichnet, dass**
der Grundkörper biodegradierbare Ankerelemente (38) ausbildet.

2. Implantat nach Anspruch 1, **dadurch gekennzeichnet, dass** der Grundkörper (12) zumindest bereichsweise aus einer biodegradierbaren Magnesium-, Eisen- oder Wolframlegierung besteht.

3. Implantat nach Anspruch 2, **dadurch gekennzeichnet, dass** die Magnesiumlegierung eine Legierung vom Typ WE ist.

4. Implantat nach Anspruch 3, **dadurch gekennzeichnet, dass** die Magnesiumlegierung eine Legierung vom Typ WE43 ist.

5. Implantat nach Anspruch 2, **dadurch gekennzeichnet, dass** die Magnesiumlegierung einen Gehalt von 0,1 Gew.% bis 10 Gew.% Aluminium enthält.

6. Implantat nach Anspruch 2, **dadurch gekennzeichnet, dass** die Magnesiumlegierung einen Gehalt von 0,01 Gew.% bis 2 Gew.% Zirkonium enthält.

7. Implantat nach Anspruch 2, **dadurch gekennzeichnet, dass** die Magnesiumlegierung ein oder mehrere Legierungsbestandteile aus der Gruppe Seltenerdmetalle, Yttrium, Lithium, Aluminium und Zirkonium enthält.

8. Implantat nach einem der vorhergehenden Ansprüche, **dadurch gekennzeichnet, dass** der Grundkörper (12) des Implantats (10) so ausgebildet ist, dass er einen ersten, nicht-expandierten Zustand und einen zweiten, expandierten Zustand einnehmen kann.

9. Implantat nach Anspruch 1, **dadurch gekennzeichnet, dass** der Grundkörper (12) rohrförmig oder zylindrisch ist.

10. Implantat nach einem der vorhergehenden Ansprüche **dadurch gekennzeichnet, dass** der Grundkörper (12)
- an seinen dem Gefäß zugewandten Seiten zumindest bereichsweise eine Beschichtung (24) und/oder
- eine oder mehrere Kavität/Kavitäten (26) und/oder
- einen oder mehrere Hohlkörper (28)
aufweist, die den Wirkstoff (22) enthalten.

11. Implantat nach Anspruch 10, **dadurch gekennzeichnet, dass** die Kavität/Kavitäten (26) von einer dem Gefäß zugewandten Seite zugänglich ist/sind, um so das Ausspülen des Wirkstoffes (22) aus dem Inneren der Kavität/Kavitäten (26) zu ermöglichen.

12. Implantat nach Anspruch 11, **dadurch gekennzeichnet, dass** sich der Wirkstoff (22) in Reinform oder in eine Matrix eingebunden im Inneren der Kavität/Kavitäten (26) befindet.

13. Implantat nach Anspruch 12, **dadurch gekennzeichnet, dass** die Matrix Hyaluronsäure oder eines ihrer Derivate ist.

## Claims

1. An implant (10) for releasing an active substance (22) into a vessel through which a bodily medium flows, wherein the implant (10) comprises a main body (12), which consists of a biodegradable material as carrier of the active substance (22) to be released in the vessel interior and to be entrained by the bodily medium and around which the bodily medium flows internally,
**characterised in that**
the main body forms biodegradable anchor elements (38).

2. The implant according to Claim 1, **characterised in that** the main body (12) consists at least in regions of a biodegradable magnesium, iron or tungsten alloy.

3. The implant according to Claim 2, **characterised in that** the magnesium alloy is an alloy of type WE.

4. The implant according to Claim 3, **characterised in that** the magnesium alloy is an alloy of type WE43.

5. The implant according to Claim 2, **characterised in that** the magnesium alloy contains a content from 0.1 % by weight to 10 % by weight of aluminium.

6. The implant according to Claim 2, **characterised in that** the magnesium alloy contains a content from 0.01 % by weight to 2 % by weight of zirconium.

7. The implant according to Claim 2, **characterised in that** the magnesium alloy contains one or more alloy constituents from the group of rare earth metals, yttrium, lithium, aluminium and zirconium.

8. The implant according to one of the preceding claims, **characterised in that** the main body (12) of the implant (10) is formed such that it can assume a first, unexpanded state and a second, expanded state.

9. The implant according to Claim 1, **characterised in that** the main body (12) is tubular or cylindrical.

10. The implant according to one of the preceding claims, **characterised in that** the main body (12) has,
- on the sides thereof facing the vessel, a coating (24) at least in regions and/or
- one or more cavity/cavities (26) and/or
- one or more hollow bodies (28),
which contain the active substance (22).

11. The implant according to Claim 10, **characterised in that** the cavity/cavities (26) is/are accessible from a side facing the vessel in order to thus allow the active substance (22) to flush out from the interior of the cavity/cavities (26).

12. The implant according to Claim 11, **characterised in that** the active substance (22) is located in the interior of the cavity/cavities (26) in pure form or is incorporated into a matrix.

13. The implant according to Claim 12, **characterised in that** the matrix is hyaluronic acid or a derivative thereof.

## Revendications

1. Implant (10) pour la libération d'une substance active (22) dans un vaisseau traversé par un fluide corporel, l'implant (10) comprenant un corps de base (12) constitué d'un matériau biodégradable, en tant que support de la substance active (22) à libérer à l'intérieur du vaisseau et à transporter par le fluide corporel, et submergé intérieurement par le fluide corporel,
**caractérisé en ce que**
le corps de base forme des éléments d'ancrage (38) biodégradables.

2. Implant selon la revendication 1, **caractérisé en ce que** le corps de base (12) est au moins par endroits constitué d'un alliage biodégradable de magnésium, de fer ou de tungstène.

3. Implant selon la revendication 2, **caractérisé en ce que** l'alliage de magnésium est un alliage du type WE.

4. Implant selon la revendication 3, **caractérisé en ce que** l'alliage de magnésium est un alliage du type WE43.

5. Implant selon la revendication 2, **caractérisé en ce que** l'alliage de magnésium contient une teneur de 0,1% en poids à 10% en poids d'aluminium.

6. Implant selon la revendication 2, **caractérisé en ce que** l'alliage de magnésium contient une teneur de 0,01 % en poids à 2% en poids de zirconium.

7. Implant selon la revendication 2, **caractérisé en ce que** l'alliage de magnésium contient un ou plusieurs composants d'alliage issus du groupe comprenant des métaux de terres rares, l'yttrium, le lithium, l'aluminium et le zirconium.

8. Implant selon l'une des revendications précédentes, **caractérisé en ce que** le corps de base (12) de l'implant (10) est conçu de manière à pouvoir être dans un premier état non-dilaté et dans un deuxième état dilaté.

9. Implant selon la revendication 1, **caractérisé en ce que** le corps de base (12) est tubulaire ou cylindrique.

10. Implant selon l'une des revendications précédentes, **caractérisé en ce que** le corps de base (12) comporte
- au moins par endroits un revêtement (24) sur ses faces tournées vers le vaisseau, et/ou
- une ou plusieurs cavité/cavités (26) et/ou
- un ou plusieurs corps creux (28)
lesquels contiennent la substance active (22).

11. Implant selon la revendication 10, **caractérisé en ce que** la/les cavité(s) (26) est/sont accessible(s) par un côté tourné vers le vaisseau, pour ainsi permettre l'évacuation de la substance active (22) à partir de l'intérieur de la/des cavité(s) (26).

12. Implant selon la revendication 11, **caractérisé en ce que** la substance active (22) se trouve sous forme pure ou intégré dans une matrice à l'intérieur de la/des cavité(s) (26).

13. Implant selon la revendication 12, **caractérisé en ce que** la matrice est un acide hyaluronique ou l'un de ses dérivés.
